# EUROPEAN PATENT SPECIFICATION

(11) **EP 1 841 361 B1**
(45) Date of publication and mention of the grant of the patent: **12.03.2014**
(21) Application number: 05855198.7
(22) Date of filing: 22.12.2005
(51) Int. Cl.: A61B 6/00, G06T 5/00

(54) **SYSTEM FOR INSERTING A NEEDLE INTO A BLOOD VESSEL**
SYSTEM ZUR EINFÜHRUNG EINER NADEL IN EIN BLUTGEFÄSS
SYSTEME D'INSERTION D'UNE AIGUILLE DANS UN VAISSEAU SANGUIN

(30) Priority: 03.01.2005 US 28467
(43) Date of publication of application: 10.10.2007
(60) Divisional application: 11186489.8
(73) Proprietor: Syris Scientific LLC, Gray ME 04039 (US)
(72) Inventor: MARCOTTE, Ronald, New Gloucester, ME 04260 (US); ARSENAULT, Mark, Sanford, MA 04073 (US); HEBOLD, Walter, Raymond, ME 04071 (US); PELLETTER, Dominic, Raymond, ME 04071 (US); WANER, Milton, New York, NY 10128 (US); FINK, Louis, Little Rock, AR 72212 (US)
(74) Representative: Bublak, Wolfgang
(86) International application number: PCT/US2005/046598
(87) International publication number: WO 2006/073869

(56) References cited:
- US-A- 5 519 208
- US-A- 5 608 210
- US-A- 6 032 070
- US-A1- 2002 115 922
- US-A1- 2003 018 271
- US-A1- 2003 210 810
- US-A1- 2004 147 836
- US-B1- 6 178 340
- ZHAROV VLADIMIR P ET AL: "Infrared imaging of subcutaneous veins." LASERS IN SURGERY AND MEDICINE 2004, vol. 34, no. 1, 2004, pages 56-61, XP002544030 ISSN: 0196-8092
- DAVIES E R: "Machine Vision: Theory, algorithms, Practicalities, PASSAGE" MACHINE VISION: THEORY, ALGORITHMS, PRACTICALITIES, XX, XX, 1 January 1997 (1997-01-01), pages 41-43, XP001091532
- LOVHOIDEN G ET AL: "Commercialization of vein contrast enhancement", PROCEEDINGS OF SPIE, S P I E - INTERNATIONAL SOCIETY FOR OPTICAL ENGINEERING, US, vol. 4958, 1 January 2003 (2003-01-01), pages 189-200, XP002362011, ISSN: 0277-786X, DOI: 10.1117/12.476117

## Description

The present invention relates generally to the non-invasive viewing of surface and subsurface blood vessels by use of an infrared imaging system. In particular, the present invention relates to guiding the insertion of a needle into a subcutaneous blood vessel with the aid of an infrared imaging system.

### BACKGROUND OF THE INVENTION

Intravenous (IV) access is the single most frequently performed invasive medical procedure in the world today. Though IV is generally considered routine, there are a number of situations in which inhibited IV access can be painful, traumatic, or even dangerous to patients. These include conditions in which subcutaneous blood vessels are difficult to locate because of patient characteristics or environmental conditions. For example, in battlefield conditions, where lighting is limited, it may be difficult - if not impossible - to locate subsurface blood vessels for injection. Easy IV access is especially critical in emergency situations in which a patient's life may depend on immediate IV access and "first-stick" accuracy.

Medical practitioners often encounter difficulty in gaining IV access in a significant portion of the patient population for which subsurface blood vessels are obscured. Such patients include obese patients, darkly pigmented patients, neonates (infants from birth to four weeks of age), children under four years of age, patients experiencing lowered blood pressure, patients who have collapsed veins, and patients requiring IV access in a minor or obscured blood vessel. Difficulties arising in these populations are demonstrated by the numbers: first-stick success rates in children and infants are currently 30%, which indicates that for 70% of the time, IV access in these populations requires more than one stick attempt. In neonates, more than 90% of IV catheters must be removed prematurely, mainly because of the improper placement of the catheters. Difficulties with IV access are encountered not only in locating the subsurface blood vessels, but also in complications that arise from improper insertion of needles or catheters in target blood vessels. Such complications include infiltration, thrombophlebitis, and infection of the IV access site.

It should be noted that children who have obscured blood vessels might lie in operating rooms for longer than 30 minutes, while medical practitioners attempt to find a blood vessel suitable for successful IV access. With the cost of operating room time approximately $14,000 per hour, delayed IV access can significantly increase the expense of both operating and office-based medical procedures.

IV access is especially critical in emergency situations when first stick accuracy can be life saving. A loss of time or inability to obtain IV access can mean the difference between life and death or, at a minimum, cause significant physical and psychological trauma. Further complicating matters, loss of patient blood and blood pressure in trauma situations can make locating subsurface blood vessels extremely difficult.

In cases where catheters, cannulas, and/or IV drips are used in patient treatment, these devices typically remain in a patient's blood vessel for a long period of time. However, in order to prevent infection, the devices are generally relocated to new body areas every 48 to 72 hours. Constant relocation of these devices over a long-term hospital stay may result in a need for medical practitioners to access less-optimal blood vessels, after more prominent blood vessels have been used. Often, these less prominent blood vessels can not easily be found by visual and tactile clues, and accessing them may require multiple sticks to the patient, which thereby causes the patient physical and emotional pain and trauma. Inhibited IV access can also subject medical practitioners to legal liability risk, by contributing to the complications associated with improper, ineffective, or delayed IV access.

IV location and access is both a visual and a tactile process. Traditional methods of IV location and access rely on the medical practitioner using his/her eyes and both hands to clean the target area, apply a tourniquet, locate the blood vessel by palpating the target area, and apply the hypodermic needle. For the sake of safe and efficient patient treatment, it is critical that the hands and eyes of the medical practitioner gaining IV access not be hindered in any way.

Medical practitioners gain proficiency at IV location and access through a process of learning and continued practice. To ensure a high standard of healthcare and patient safety, it is imperative that medical practitioners do not attempt to gain IV access before they are adequately trained. Unfortunately, traditional methods of IV location and access may require years of trial-and-error practice and thereby delay critical healthcare, which increases healthcare costs and possibly jeopardizes patient health. Any advancement in healthcare practices that reduces the amount of training time required for proficiency in gaining skill at IV access could contribute significantly to improved patient care.

In addition to efficiency concerns, in cases where catheters are inserted in order to dispense media such as chemotherapy drugs, saline solutions, or other potentially harmful substances into the blood vessel, it is a common practice to insert the catheter and then X-ray, CT scan, or use other common medical imaging techniques on the insertion site, to insure that the catheter is properly inserted. This process adds both to the time required to complete the procedure as well as the overall cost thereof.

In order to provide the highest standard of care while reducing the cost of healthcare, it is imperative that medical practitioners locate and gain access to subsurface blood vessels, and insure proper insertion thereof, in a rapid and accurate manner. Simplified IV location and access can help to save lives in emergency situations, avoid the trauma of multiple sticks in situations in which patients' vessels are difficult to locate, reduce the number of complications that stem from improperly inserted hypodermic needles and IVs, and reduce costs of medical procedures, by speeding up a critical bottleneck in many medical procedures: IV access. Therefore, what is needed is a hands-free device that allows medical practitioners to rapidly and accurately locate subsurface blood vessels for IV access.

As of late, apparatus have developed that help medical personnel more accurately locate blood vessels. For example a system and method for locating subcutaneous blood vessels via IR enhancement is described in U.S. Patent No. 4,817,622, entitled, "Infrared imager for viewing subcutaneous location of vascular structures and method of use," in which a human appendage, typically the inside of the elbow, is illuminated with an IR source, for example, at least one incandescent light bulb. A video camera for producing a video image and immediately overlying monitor for displaying the video image is utilized to look at the flesh. The camera is sensitive to IR radiation. A video display in which IR absorbing or scattering contrasting portions of the flesh are highlighted, for example, hard-to-find veins for inserting needles. A contrast enhancing circuit is included, which discloses amplifying the video information with high contrast enhancement of the video. Adaptation of the disclosed circuit to conventional TV charge coupled device cameras and monitors is illustrated with compensation of horizontal sweep to even image background, intensity averaging line-to-line for vertical image uniformity, and display of image contrasts, in a log amplification format. While the '622 patent describes an IR blood vessel viewer, the '622 patent utilizes an analog signal processor, which is not adequate for supporting the digital algorithms needed for true image enhancement and visualization.

More recently, U.S. patent 5,519,208, purportedly describes a method and apparatus for gaining intravenous access that includes a source of radiation for irradiating an area of the patient with radiation having a wavelength that is absorbed in areas containing veins and reflected in all other areas. The reflected radiation is then read and the output displayed. Using this technique, venous structures appear as dark lines on the display, enabling a user to position the tip of a hypodermic needle at an appropriate location for drawing blood.

Along similar lines, U. S. Patent 6,032,070, purports to describe a system and method to view an anatomical structure such as a blood vessel in high contrast with its surrounding tissue. The system and method may be used to produce an image of an anatomical structure using reflected electromagnetic radiation singularly scattered from target tissue. The system and method purport to provide improved contrast between any anatomical structure and its surrounding tissue for use in any imaging system.

Likewise, U.S. Patent 6,230,046, purportedly discloses a system and method for enhancing visualization of veins, arteries or other subcutaneous natural or foreign structures of the body and for facilitating intravenous insertion or extraction of fluids, medication or the like in the administration of medical treatment to human or animal subjects. The system and method include a light source for illuminating or transilluminating the corresponding portion of the body with light of a selected wavelengths and a low-level light detector such as night vision goggles, a photomultiplier tube, photodiode or charge coupled device for generating an image of the illuminated body portion, and optical filter(s) of selected spectral transmittance which can be located at the light source(s) detector, or both.

Document US6178340 discloses a three-dimensional infrared imager assisting a subcutaneous puncture. A double image is superimposed on the viewer and the user wears blue-red eyeglasses to create the three-dimensional image from the double image on the viewer. With the depth perception provided by the three-dimensional image, the user can accurately penetrate the vein on the initial attempt, thereby lowering the incidence of medical complications associated with inaccurate puncture. A liquid crystal display (LCD) mounted in a frame is provided on the upper surface of the apparatus. Sensitive, charge-coupled devices are provided which develop images real-time and deliver them to a microprocessor which synchronizes the images and allows the user to manipulate the images as desired, controlling effects such as brightness, contrast, sharpness and and edge enhancement.

The above referenced patents are illustrative of attempts to demarcate blood vessels from surrounding tissue. The systems and methods of the described patents are non-invasive and, most importantly, provide the near "real time" visualization of the image necessary for these devices to serve their practical purpose. However, because of the need to provide near "real time" images, these devices primarily depend on raw images, or images marginally enhanced by traditional analog means, which are of relatively poor quality for venepuncture accuracy. Therefore, there is a need not only for a device for visualizing subsurface blood vessels, but also a system and method for vascular image location, image enhancement, and hands-free manipulation, for quick and accurate IV access.

Therefore, there is a need for an improved system and method for locating and accessing a target blood vessel that that has the vein enhancing features of the prior art devices discussed above, but produces high quality images in near "real time" such that the system may be used by medical personnel during venepuncture, that allows target blood vessels to be more accurately and rapidly located than is possible using current systems and methods, that allows target blood vessels to be more easily located in difficult conditions and body types (e.g., obese patients, dark pigmentation skin, neonates, collapsed veins, low lighting), that reduces patient pain and trauma, both emotionally and physically, that allows minimally trained medical staff to provide IV access, and which provides a rapid confirmation that the needle is properly inserted without the use of X-rays, CT scans or other common medical imaging equipment.

### SUMMARY OF THE INVENTION

The present invention is a needle insertion system for inserting a needle within a blood vessel.

The invention is defined in the independent claim 1. Preferred embodiments are defined in the dependent claims.

In its most basic form, the needle insertion system includes an imaging system and a needle having a needle body and a needle tip. The imaging system includes at least one infrared emitter an infrared detector, a computing unit, a display device, and a power source. In operation a user views the display of the imaging system and locates a target blood vessel, align the needle body with the target blood vessel, pierces the blood vessel with the needle tip, introduces the needle into the target blood vessel, advances the needle until the display of the imaging system shows that a sufficient depth has been reached, performs a medical procedure, and withdraws the needle from the blood vessel.

The infrared emitter is, or emitters are, configured to illuminate a region under a surface of skin with waves of infrared light. The infrared detector, preferably a CMOS camera, is configured to accept waves of infrared light reflected from the region under the surface of the skin and includes an output for outputting a signal corresponding to unenhanced image data. The computing unit includes an input for accepting the unenhanced image data, a memory, means for enhancing and outputting result images in which enhanced images of blood vessels are shown within the images of the region under the surface of the skin, and an output for outputting the enhanced images in substantially real time. The display device inputs the enhanced images from output of the computing unit and displays the enhanced images. Finally, the power source is in electrical communication with the infrared emitter, the infrared detector, the computing unit and the display device and provides power thereto.

In the imaging system, the means for enhancing and outputting result images includes a digital signal processing unit programmed with computer program means for enhancing and outputting result images at a rate of at least five frames per second. The preferred computer program means includes program means for Gaussian blurring a raw image with a kernel radius of 15, program means for adding an inverse Gaussian-blurred image to the raw image, and program means for level adjusting a result image to use an entire dynamic range.

The imaging system includes a headset to which the two arrays of infrared emitters, infrared detector, computing unit, display, and power source are attached. The headset preferably includes a pair of extension arms extending therefrom and a mounting surface pivotally attached to the pair of extension arms. In this arrangement, the two arrays of light emitting diodes and the infrared detector are attached to the mounting surface. The display is disposed upon the headset such that a user is able to view both the display and the surface of the skin without removing the headset.

The preferred light emitting diodes are surface-mounted light emitting diodes comprising integral micro reflectors. At least one light shaping diffuser is preferably disposed between the arrays of surface mounted light emitting diodes and the surface of the skin. Such a diffuser is preferably integral to the light emitting diodes, but may be a separate diffuser. At least one first polarizing filter is preferably disposed between the surface mounted light emitting diodes and the surface of the skin, and at least one second polarizing filter is preferably disposed between the surface of the skin and the infrared detector. The polarizing filters preferably act to cross polarize the light, but may provide any arrangement ofpolarization, or be eliminated completely.

The infrared detector is preferably a CMOS camera adapted to generate digital data corresponding to the waves of infrared light reflected from the subcutaneous blood vessels located in the region under the surface of the skin. The CMOS camera may include a high band pass filter adapted to filter out substantially all light outside of an infrared spectrum, or may be adapted to receive both infrared and visible spectrum light. A camera lens is preferably disposed between the surface of the skin and the CMOS camera in order to adjust the focal length of the image. However, in embodiments in which a specialized CMOS camera having the proper focal length is used, or those in which the images are digitally adjusted for proper visualization on the display unit, the camera lens is eliminated altogether.

The display is an LCD screen type display having a pair of LCD screens. At least one optical lens is disposed between the LCD screens and a pair of eyes of a user to adjust for differences between the enhanced image an the unenhanced image viewed directly the user.

The preferred computing unit includes a digital signal processing unit and image data storage means for storing a multiple images for future viewing. The computing unit also includes an interface for inputting data from a data input and outputting data to a data output device.

As used in connection with the present invention, the term "needle" refers to any object that is used to puncture and penetrate the skin during a medical procedure, and specifically includes commonly used needles such as hypodermic needles and catheter needles. The preferred needle is a catheter needle and, therefore, the following description focuses upon embodiments utilizing a catheter. Notwithstanding this fact, it is recognized that the system is applicable to any type of needle, and not merely catheters, and that the use of the term "catheter" and "catheter needle" should be interpreted as encompassing any type of needle, as defined herein.

The needle preferably includes at least one substance disposed thereon, or therein, for enhancing a visibility of the needle body by said imaging system when compared with the visibility of the needle without the aid of the substance. In embodiments in which a traditional metal needle is used, this substance is preferably and IR-opaque or IR-reflecive substance disposed upon an outer surface of the needle body. Regardless of which substance is used, it is preferably disposed in a pattern that further enhances the visibility of the needle body over those upon which such a pattern is not disposed. In still other embodiments, both an IR-opaque and an IR reflective substance are used and are disposed thereon in a pattern that further enhances the visibility of the needle body over those upon which such a pattern is not disposed. Preferred patterns include zebra stripes, "figure eights" and measured graduations, which may be used as a further aid in gauging the depth of penetration of the needle.

In some embodiments, the needle is a plastic needle manufactured of a molded plastic material, the substance is preferably disposed within the plastic material during manufacture. As was the case with the traditional metal needles described above, the substance may be an IR-opaque substance, and IR-reflective substance or a combination of both and, that substance may be likewise be disposed within the plastic material in a pattern that further enhances the visibility of the needle by the system. The needle insertion system of claim 12 wherein one of said at least one substance for enhancing a visibility of said needle body is an IR opaque substance.

Therefore, it is an aspect of the invention to provide an improved system for locating and accessing a target blood vessel that that produces high quality images in near "real time" such that the system may be used by medical personnel during venepuncture.

It is a further aspect of the invention to provide an improved system for locating and accessing a target blood vessel that allows target blood vessels to be more accurately and rapidly located than is possible using current systems and methods.

It is a further aspect of the invention to provide an improved system for locating and accessing a target blood vessel that allows target blood vessels to be more easily located in difficult conditions and body types (e.g., obese patients, dark pigmentation skin, neonates, collapsed veins, low lighting).

It is a further aspect of the invention to provide an improved system for locating and accessing a target blood vessel that reduces patient pain and trauma, both emotionally and physically.

It is a further aspect of the invention to provide an improved system for locating and accessing a target blood vessel that allows minimally trained medical staff to provide IV access.

It is a still further aspect of the invention to provide an improved system for locating and accessing a target blood vessel that provides a rapid confirmation that the needle is properly inserted without the use of X-rays, CT scans or other common medical imaging equipment.

These aspects of the invention are not meant to be exclusive and other features, aspects, and advantages of the present invention will be readily apparent to those of ordinary skill in the art when read in conjunction with the following description, appended claims and accompanying drawings.

### BRIEF DESCRIPTION OF THE DRAWINGS

FIG. 1 is a front isometric view of the preferred embodiment of the system of the present invention.
FIG. 2 is a rear isometric view of the preferred embodiment of the system of the present invention.
FIG. 3 is an isometric view of the preferred embodiment of the system worn on the head of a user.
FIG. 4 is a diagram illustrating the operation of one embodiment of the infrared imaging system of the present invention to detect subcutaneous blood vessels.
FIG. 5A is an image of a human forearm showing unpolarized visible spectrum light reflected from the forearm and captured by a camera.
FIG. 5B is a raw image of the human forearm of FIG. 5A showing cross-polarized infrared spectrum light reflected from the forearm and captured by the CMOS camera of the preferred system of the present invention.
FIG. 5C is an enhanced image resulting from the operation of the computer program product of the present invention on the raw image of the human forearm of FIG. 5B.
Figure 6 illustrates an exploded view of a catheter with the catheter needle withdrawn from the cannula in accordance with the invention.
Figure 7 is a flow diagram of using the system to aid in locating and inserting a catheter into a blood vessel in accordance with the invention.

### DETAILED DESCRIPTION OF THE INVENTION

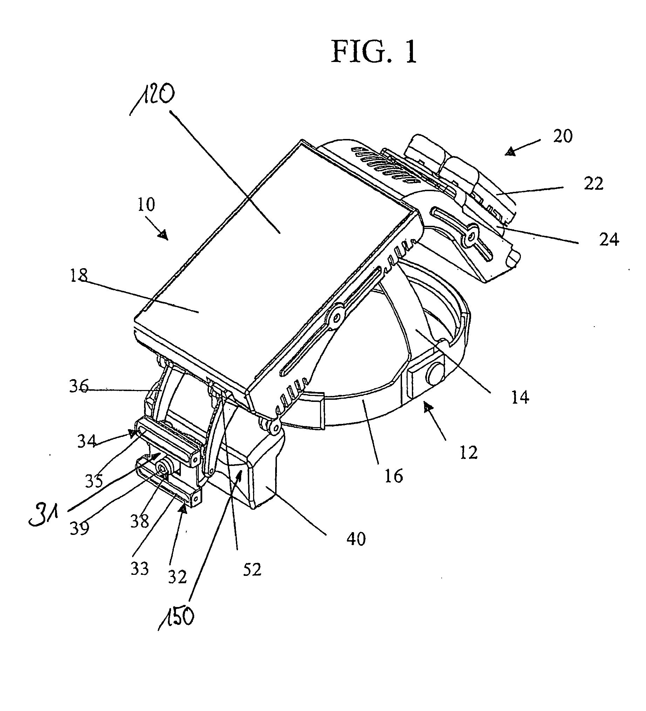

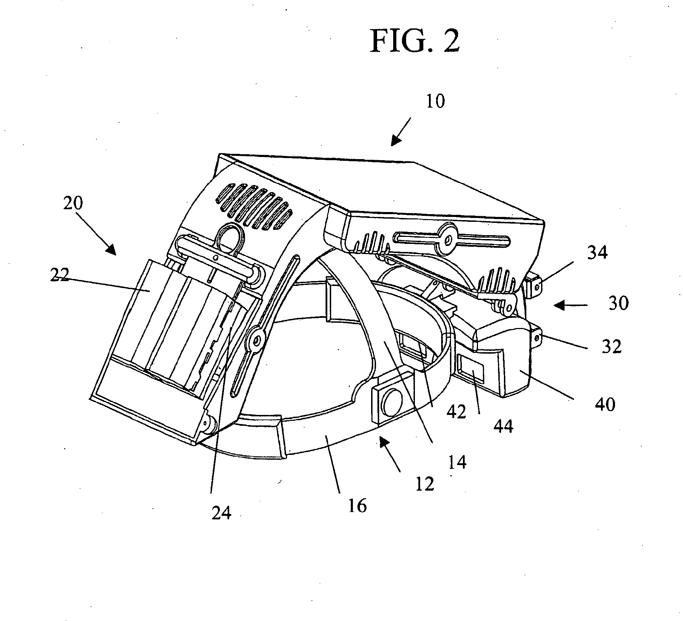

FIGS. 1 - 3 show the preferred embodiment of the imaging system 10 that forms a part of the needle insertion system of the present invention. The imaging system 10 includes a headset 12 to which all system components are attached. The preferred headset 12 includes two plastic bands 14, 16; a vertical band 14 connected to sides of a horizontal band 16. The vertical band 14, holding most of the system components, generally acts as a load-bearing member, while the horizontal band 16 is adjustable such that it snugly fits about the forehead of the person using the system.

A pivoting housing 18 is attached to the headband 12. The housing 18 is substantially hollow and is sized to house and protect a headset electronics unit 120 disposed therein. Attached to the housing 18 are a power supply 20, an image capture assembly 30, and an enhanced image display unit 40.

The power supply 20 for the headset electronics unit 120 preferably includes two rechargeable lithium ion batteries 22, which are connected to the electronics unit via a pair of battery terminals 24 attached to the rear of the housing 18. The rechargeable lithium ion batteries 22 are preferably of the same type commonly used with video camcorders, as these are readily available, are rechargeable without fear of memory problems, make the unit completely portable, and will provide sufficient power to the headset electronics unit 120 when two such batteries 22 are used. However, it is recognized that any power supply 20 known in the art to supply power to electronics, such as alternating current power plugs, may be employed to achieve similar results.

The image capture assembly 30 is powered thorough the headset electronics unit 120 and includes a pair of infrared emitters 32, 34, and a camera 38, or other infrared detector, disposed between the infrared emitters 32, 34. The infrared emitters 32, 34 and camera 38 are attached to a common mounting surface 31 and are pivotally connected to a pair of extension arms 36 that extend from the housing 18. Mounting in this manner is preferred as it allows the emitters 32, 34 and camera 38 to be aimed at the proper target, regardless of the height or posture of the person wearing the headset. However, it is recognized that both could be fixedly attached to the headset, provided the relationship between the emitters 32, 34 and camera 38 remained constant.

The infrared emitters 32, 34 of the preferred embodiment are surface mount LEDs (light emitting diodes) that feature a built-in micro reflector. Light emitting diodes are particularly convenient when positioned about the head because they are found to generate less heat then conventional bulbs and do not require frequent changing. Further, surface mount LED's that emit infrared light through light shaping diffusers to provide uniform light and are readily adapted for attachment to a variety of other flat filter media. The preferred infrared emitters 32, 34 each utilize a row, or array, of such LED's in front of which is disposed a light shaping diffuser (not shown). Such emitters 32, 34 may be purchased from Phoenix Electric Co., Ltd., Torrance, CA.. First polarizing filters 33, 35 are mounted in front to the light shaping diffusers of each of the infrared emitters 32, 34. These polarizing filters 33, 35 are preferably flexible linear near-infrared polarizing filters, type HR, available from the 3M Corporation of St. Paul, Minnesota. In operation, the LED's are powered through the headset electronics unit 120 and emit infrared light, which passes through the light shaping diffuser 205 and the first polarizing filters 33, 35 to produce the polarized infrared light 215 that is directed upon the object to be viewed. In some embodiments, a first plurality of infrared emitters 32, 34 are provided to produce light having a wavelength within a range suitable for viewing blood vessels and a second plurality of infrared emitters 32, 34 are provided to produce light having a wavelength within a range suitable for viewing the needle.

The camera 38 is adapted to capture the infrared light 230 reflected off of the object to be viewed and to provide this "raw image data" to the headset electronics unit 120. The preferred camera 38 is a monochrome CMOS camera that includes a high pass filter (not shown) that filters out all light outside of the infrared spectrum, including visible light. A CMOS camera is preferred as it produces pure digital video, rather than the analog video produced by the CCD cameras disclosed in the prior art, and is, therefore, not susceptible to losses, errors or time delays inherent in analog to digital conversion of the image. The CMOS camera is may be any number of such cameras available on the market, including the OMNIVISION® model OV7120, 640 X 480 pixel CMOS camera, and the MOTOROLA® model XCM20014. In the test units, the OMNIVISION® camera was used with good success. However, it is believed that the MOTOROLA® camera will be preferred in production due to its enhanced sensitivity to infrared light and the increased sharpness of the raw image produced thereby.

A camera lens 240 is preferably disposed in front of the camera 38. This camera lens 240 is preferably an optical lens that provides an image focal length that is appropriate for detection by the camera 38, preferably between six inches and fourteen inches, eliminates all non-near IR light, and reduces interference from other light signals. The preferred camera lens 240 is not adjustable by the user. However, other embodiments of the invention include a camera lens 240 that may be adjusted by the user in order to magnify and/or sharpen the image received by the camera 38. Still others eschew the use of a separate camera lens 240 completely and rely upon the detection of unfocused light by the camera 38, or other infrared detector.

A second linear polarizing filter 39 is disposed in front of the lens 240 of the camera 38. This second polarizing filter 39 is preferably positioned so as to be perpendicular to the direction of polarization through the first polarizing filters 33, 35 in front of the infrared emitters 32, 34, effectively cross polarizing the light detected by the camera 38 to reduce spectral reflection. The polarizing filter 39 was selected for its high transmission of near-infrared light and high extinction of cross-polarized glare. Such polarizer may be purchased from Meadowlark Optics, Inc. of Frederick, Colorado under the trademark VERSALIGHT®.

The camera 38 is in communication with the headset electronics unit 120 and sends the raw image data to the unit for processing. The headset electronics unit includes the electronics required to supply power from the power supply 20 to the image capture assembly 30, and an enhanced image display unit 40, and the compatible digital processing unit 122 which accepts the raw image data from the camera 38, enhances the raw image, and sends an output of the enhanced image to the enhanced image display unit 40 and, optionally, to an interface 52. In the preferred embodiment, this interface 52 is standard VGA output 52. However, interface 52 may be any electronic data I/O interface capable of transmitting and receiving digital data to and from one or more input or output devices, such as an external monitor, external storage device, peripheral computer, or network communication path.

The preferred digital signal-processing unit 122 is a digital media evaluation kit produced by ATEME, Ltd SA, Paris, France under model number DMEK6414, which uses a Texas Instruments TMS320C6414 digital signal processor. This processing unit 122 is preferably programmed with an embodiment of the computer program means described in the applicants' co-pending United States Patent Application Serial No. 10/760,051, in order to enhance the images. The image enhancement algorithms embodied in the computer program means utilize several elemental processing blocks, including (1) Gaussian Blurring a raw image with a kernel radius of 15, (2) adding the inverse Gaussian-blurred image to the raw image, and (3) level adjusting the result to use the entire dynamic range. Image enhancement is performed in a series of steps, which are coded into a computer program that runs on digital signal processor 120. The programming languages are typically C language and assembly language native to digital signal processor 120. An example algorithm is as follows: 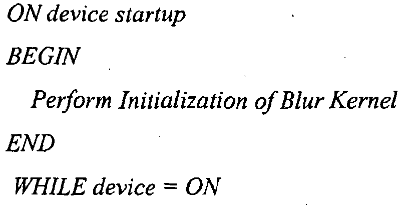 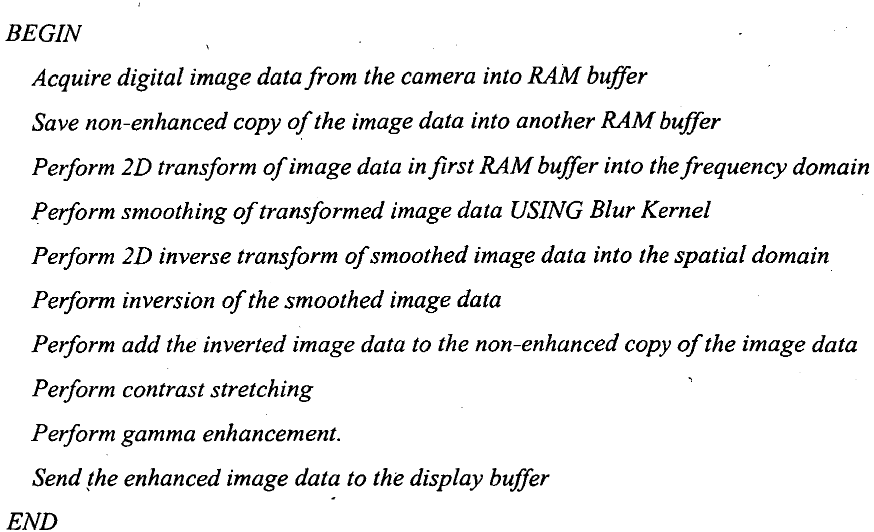 However, it is understood that other systems may use different means for similarly enhancing such images in near real-time and, therefore, it is understood that all embodiments of the invention need not include this program product or perform the methods described in the above referenced patent application.

The enhanced image is outputted from the processing unit to the enhanced image display unit 40. The preferred display unit 40 is distributed by i-O Display Systems of Sacramento, CA, under the trademark I-Glasses VGA. This display unit 40 includes a binocular display that includes a pair of LCD screens in front of which are disposed a pair of optical lenses 42, 44 that allow the focal length to be adjusted for ease of viewing. The optical lenses 42, 44 provides image depth perception compensation to the user when the system 10 is used in a bifocal mode. That is, when the user views the body target area via display 150, the optical lenses 42, 44 ensure that the image appears similarly sized and distanced as when the user views the target area without using display 40. However, it is understood that a monocular display unit 40 having no such focal length adjustment could likewise be used. The preferred display unit 40 also includes an on-screen display that is not currently used, but may be used in the future to show what enhancement option has been chosen by the user.

The system 10 may be used in a total immersion mode, in which the user focuses on the target area by using exclusively display 40. Alternatively, the system 10 may be used in a bifocal mode, in which the user views the body target area via a combination of display 40 and the naked eye. In bifocal mode, the user alternates between viewing the enhanced and non-enhanced image views of the body target area, by directing his/her gaze upward to display 40 or downward toward the body target area and away from display 150.

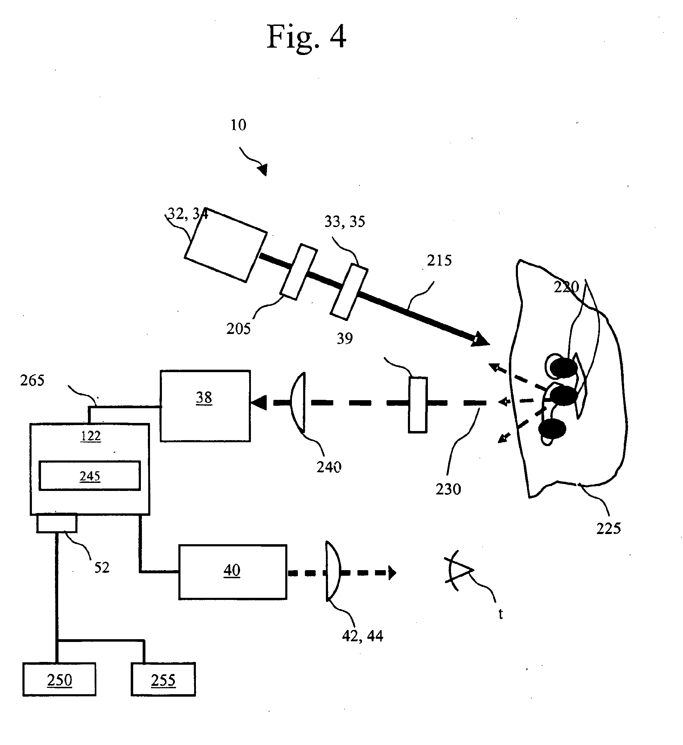

Figure 4 illustrates, an example of the infrared imaging system 10 used to view subcutaneous blood vessels 220, such as arteries, veins, and capillary beds, which are present under the surface 225 of normal human skin. The infrared imaging system 10 described in connection with FIG. 4 includes all of the features described above, in addition to including a camera lens 240, image data storage means 245, a data input 250, and data output 255.

Image data storage means 245 is any means of digital data storage that is compatible with digital signal processor 120 and may be used to store multiple enhanced and/or unenhanced images for future viewing. Examples of such image data storage are random access memory (RAM), read-only memory (ROM), personal computer memory card international association (PCMCIA) memory card, and memory stick. Depending on memory size, hundreds or thousands of separate images may be stored on the image data storage means 245.

Data output 250 is any external device upon which the image data produced by digital signal processor 120 may be viewed, stored, or further analyzed or conditioned. Examples of data output 250 devices include external video displays, external microprocessors, hard drives, and communication networks. Data output 250 interfaces with digital signal processor 120 via interface 52.

Data input 255 is any device through which the user of the system 10 inputs data to digital signal processor 122 in selecting, for example, the appropriate enhancement algorithm, adjusting display parameters, and/or choosing lighting intensity levels. Examples of data input 255 devices include external keyboards, keypads, personal digital assistants (PDA), or a voice recognition system made up of hardware and software that allow data to be inputted without the use of the user's hands. Data input 255 may be an external device that interfaces with digital signal processor 120 via interface 52, or may be integrated directly into the computing unit.

Digital data path 265 is an electronic pathway through which an electronic signal is transmitted from the camera 38 to the digital signal processor 122.

In operation, the infrared imaging system 10 is powered on and the infrared emitters 32, 34 produce the necessary intensity and wavelengths of IR light. This is preferably between 850 nm and 950 nm wavelengths, which are required to interact and reflect from oxyhemoglobin and deoxyhemoglobin contained within normal blood, but may be a different wavelength chosen to allow the needle to be viewed, or pulsed between the wavelengths required for viewing blood and the wavelengths required for viewing the needle. The resulting light path passes through diffuser system 205, where it is dispersed into a beam of uniform incident light 215 of optimal intensity and wavelength. Incident light 215 passes through first polarizers 33, 35, which provide a first plane of polarization. Polarization of incident light 215 reduces the glare produced by visible light by reflection from skin surface 225. Incident light 215 is partially absorbed by the oxyhemoglobin and deoxyhemoglobin that is contained with subcutaneous blood vessels 220 and, thus, produces reflected light 230.

Reflected light 230 passes through second polarizer 39, which provides a second plane of polarization. The second plane of polarization may be parallel, orthogonal, or incrementally adjusted to any rotational position, relative to the first plane of polarization provided by first polarizers 33, 35. Reflected light 230, passes through first lens 240, which provides an image focal length that is appropriate for detection by the camera 38, eliminates all non-near IR light, and reduces interference from other light signals.

Camera 38 detects reflected light 230 and converts it to an electronic digital signal by using CCD, CMOS, or other image detection technology. The resulting digital signal is transmitted to digital signal processor 122 via digital signal path 265. Digital signal processor 122 utilizes a number of algorithms to enhance the appearance of objects that have the spatial qualities of blood vessels, so that the user can distinguish blood vessels easily from other features when viewed on display 40. Such enhancement might include, for example, image amplification, filtering of visible light, and image analysis. The resulting digital signal is transmitted to display 40 via digital signal path 265, where it is rendered visible by LCD, CRT, or other display technology. Additionally, the resulting digital signal may be outputted to an external viewing, analysis, or storage device via interface 52. The image produced by display 40 is then corrected for depth perception by second lens 42, 44, such that, when the user views the body target area via display 40, the image appears similarly sized and distanced as when the user views the target area with the naked eye.

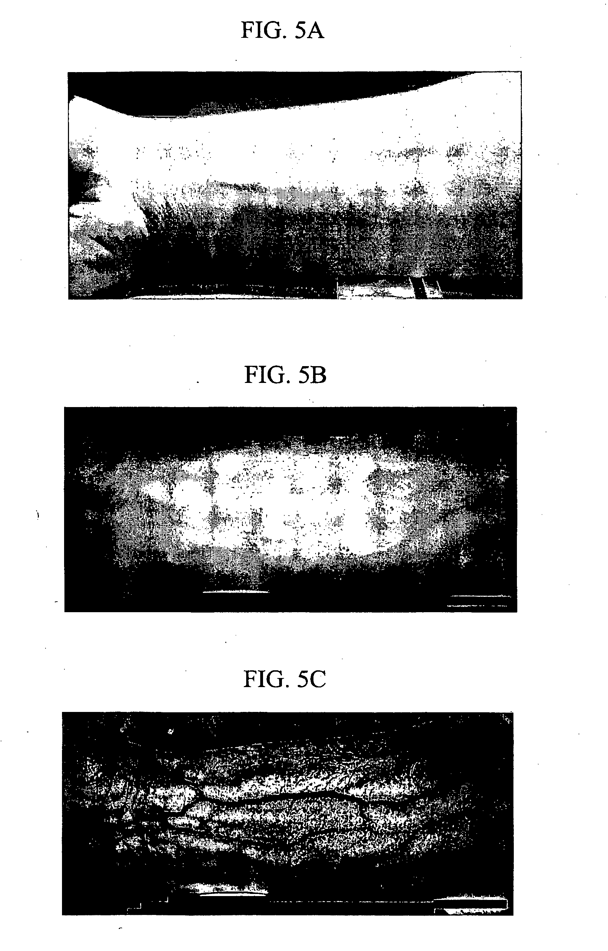

FIGS. 5A, 5B and 5C demonstrate the image enhancement produced by the system of the present invention. FIG. 5A is a photograph of a human forearm using light from the visible spectrum. As seen from this photograph, it is difficult to locate the veins upon visual inspection. FIG. 5B is a raw image of the same human forearm sent from the image capture assembly 30 of the present invention to the processing unit. The veins in this image are considerably more visible than those in FIG. 5A. However, they are not sufficiently dark and well defined to allow easy location of the veins during venepuncture. FIG. 5C is an enhanced image using the image enhancement process of the present invention. As can be seen from this figure, the veins are very dark and, therefore, are easily located for venepuncture.

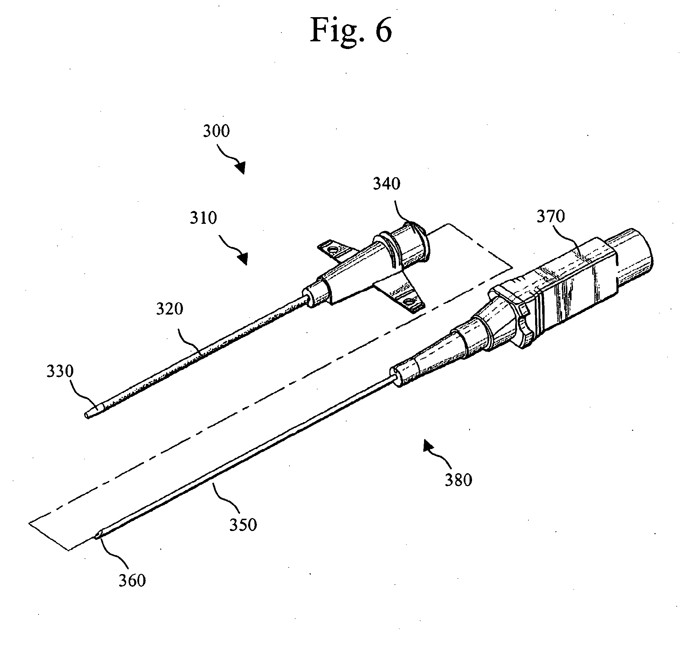

Figure 6 illustrates an exploded view of a catheter 300, with the catheter needle 350 withdrawn from cannula 310. As noted above, the catheter is a specific type of needle that may be part of the present system and the invention is not limited to catheter needles. However, due to the particular steps involved in inserting a catheter needle 350 and disposing a Cannula 310 within a blood vessel using the system of the present invention, only embodiments utilizing a catheter 300 has been described herein. Notwithstanding this fact, it is recognized that the system is applicable to any type of needle, and not merely catheters, and that the invention should not be seen as being so limited.

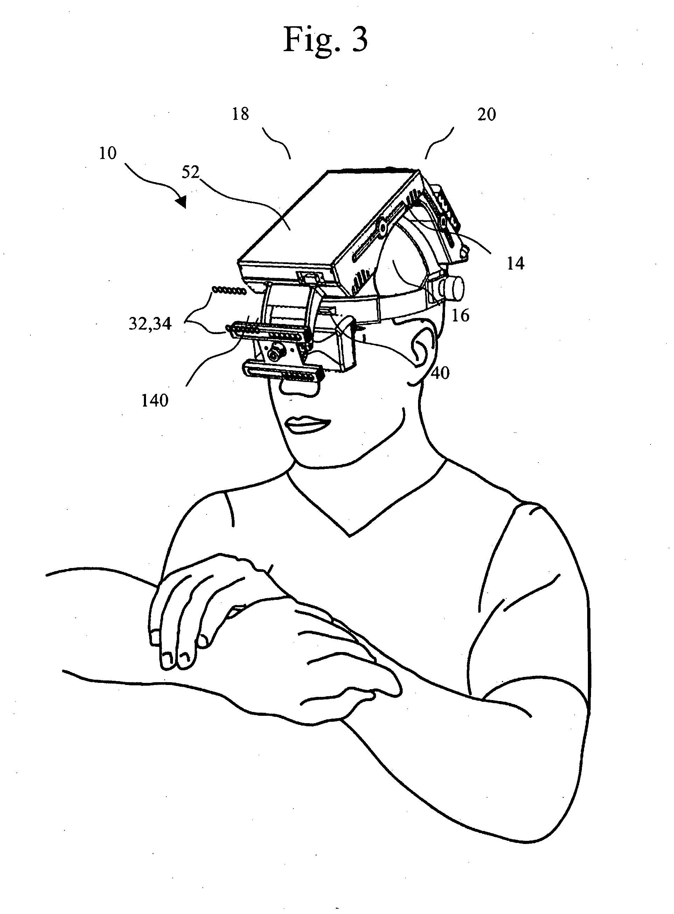

Catheter 300, described in more detail in reference to Figure 3, is referenced throughout this disclosure and is fully described and shown in U.S. patent applications US2002/0115922, US2003/0187360, and US2004/0019280. Catheter 300 includes a cannula 310, and a catheter body 380. Cannula 310 further includes a cannula sheathing 320, a cannula tip 330, and a cannula housing 340. Catheter body 380 further includes a catheter needle 350, a needle tip 360, and a flash chamber 370. An exploded view of a catheter is fully described and shown in US2004/0019280, US2003/0187360, and US2002/0115922.

Catheter 300 is an intraluminal, indwelling catheter that is well known in standard medical practice and is presented for illustrative purposes. Cannula sheathing 320 is a hollow body that is constructed, typically, of medical-grade plastic and that has an inside diameter sufficient for receiving catheter needle 350. Catheter needle 350 is a hollow needle that is sheathed with cannula sheathing 320. Needle tip 360 is the sharp proximal tip of catheter needle 360 and protrudes from cannula tip 330 a sufficient distance in order to allow for piercing of the skin. The specific distance of penetration is based upon a number of factors, including the procedure to be performed, the body type of the patient and the user's personal preference. Accordingly, a sufficient distance in this context means a distance that the user deems to be sufficient. Cannula housing 340 may receive standard intravenous tubing (not shown) in an IV catheter. Flash chamber 370 is preferably constructed of medical-grade plastic and is a hollow chamber forming the distal end of catheter body 380.

An IR-opaque or IR-reflective substance or pattern may be applied to catheter needle 350 and needle tip 360, so as to render the needle position and travel path more visible to the medical practitioner when viewed with the system 10 and, thus, assist in catheter placement. An IR-opaque substance, such as indocyanine green, may be applied to catheter needle 350 and needle tip 360. Alternatively, an IR-opaque or an IR-reflective pattern, such as solid bands, "zebra stripes," or similar strongly identifiable markings may be applied to cannula sheathing 320. The intent is to produce a pattern that is easily visualized via display 40 of the system 10 and that is distinctive from nearby anatomical structures. The IR-opaque or IR-reflective substance or pattern is applied to catheter 300 during manufacture or sometime prior to patient treatment. Alternatively, catheter 300 and/or cannula tip 330 may be illuminated by IR radiation that is provided to catheter 300 via fiber optics, micro-diodes, or other IR-emitting source. These and additional embodiments regarding catheter 300 are further disclosed in detail in U.S. patent applications US2004/0019280, US2003/0187360, and US2002/0115922.

In operation, a medical practitioner user prepares a patient's body target area for catheter 300 insertion by using standard medical practices, including, for example, cleaning the target area, and applying a tourniquet. The user puts on the headset 12, provides power to the system 10, and may optimize various parameters of the system 10, including, for example, the patient's body type, body target area, and skin pigmentation, via data input 255. The user searches for the target blood vessel by directing the infrared emitters 32, 34 onto the body target area and viewing the target area via display 40 Once the target blood vessel is located, the user looks downward from display 40 to view catheter 300 in his/her visual field. Utilizing either his/her naked eye or the IR-enhanced image that appears on display 40, the user aligns catheter 300 above and parallel to the target blood vessel and pierces skin surface 225 with needle tip 330. The user, by utilizing either his/her naked eye or the enhanced image appearing on display 40, pierces skin surface 225 with needle tip 360 and introduces the catheter 300 into the target blood vessel. When catheter 300 enters the target blood vessel, blood will flow into flash chamber 370. Because of the IR-opaque or IR-reflective substance or pattern that was previously applied to catheter needle 350, the position and travel path of catheter needle 350 is clearly visible to the user on display 40, which allows the user to guide its depth and travel path more accurately. The user advances catheter 300 into the target blood vessel, until a sufficient depth has been reached, after which catheter needle 350 and catheter body 380 are withdrawn, which leaves cannula 310 remaining in the target blood vessel. Cannula 310 is secured in place, and the procedure completed using standard medical practices.

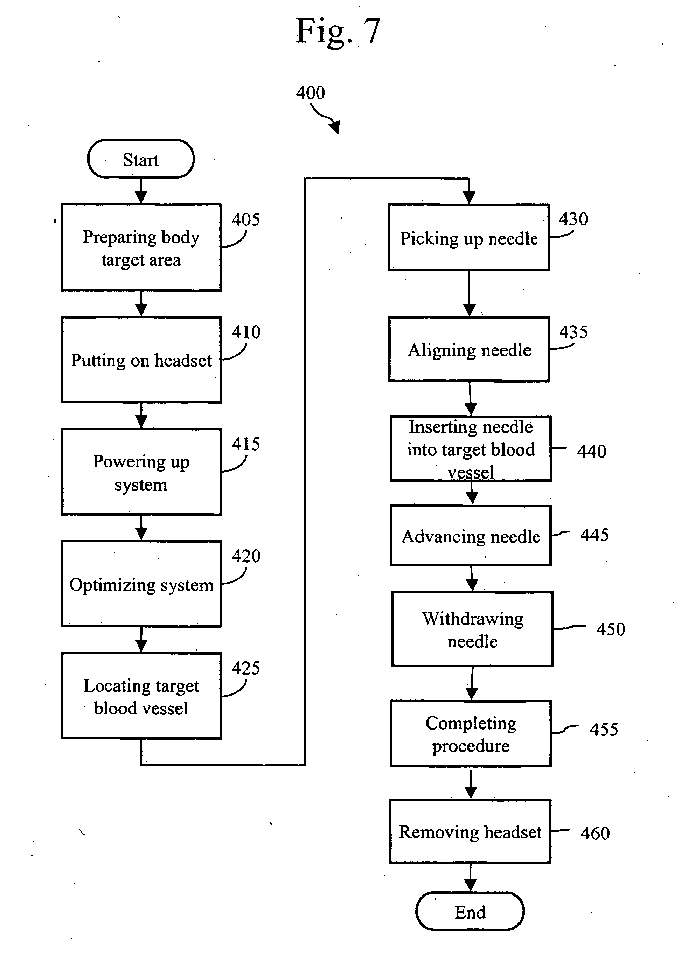

Figure 7 illustrates a flow diagram of a method 400 of using the system 10 to aid in the insertion of catheter 300 into a target blood vessel. Method 400 includes the steps of:
Step 405: Preparing body target area

In this step, a user, such as a medical practitioner (e.g., doctor, nurse, or technician), prepares the patient's body target area for injection by using standard medical practices. This might include, for example, positioning the target body area (e.g., arm), applying a tourniquet, swabbing the target area with disinfectant, and palpating the target area. Method 400 then proceeds to step 410.
Step 410: Putting on the headset 12

In this step, the user places the headset 12 on his/her head and adjusts head mount 16 for size, comfort, and a secure fit. Method 400 then proceeds to step 415.
Step 415: Powering up the system

In this step, the user powers up the system 10, by activating a switch controlling the power source 20. Method 400 proceeds to step 420.
Step 420: Optimizing the system

In this step, the user uses data input 255 to adjust various parameters of the system 10, including specifying the appropriate digital signal processor 123 algorithms according to, for example, the patient's body type, pigmentation, and age, and/or characteristics of the needle, intensity levels of the infrared emitters 32, 34, and parameters for the images to be viewed on the display 40. As noted above, the optimizing step includes the step of adjusting the operation of the infrared emitters 32, 34 to pulse between two sets of wavelengths of light in order to allow the user to view both the target blood vessel and the needle. Method 400 then proceeds to step 425.

It should be noted that Steps 405, 410, 415 and 420 may be performed in any order, e.g., the user may power up the system 10 and optimize it, prior to putting it on. Further, it is recognized that the optimizing step 420 may be eliminated altogether, with settings being preset at the factory.
Step 425: Locating target blood vessel

In this step, the user searches non-invasively for the desired target blood vessel(s) (e.g., vein, artery, or capillary bed), by directing the incident light 215 from the infrared emitters 32, 34 on the body target area, viewing the target area on display 40, and focusing the camera lens 240 on the skin surface 225. As viewed on display 40, the target blood vessel(s) will be visually enhanced, i.e., appear darker than the surrounding tissue, which enables the user to insert the catheter into the target blood vessel more accurately and rapidly. Because of the hands-free operation of the system 10, the user is free to handle the body target area with both hands, for stability, further palpation, and cleansing, for example. Using the system 10 in a bifocal mode, the user may look down from display 40 to see the body target area as it appears under normal, non-enhanced conditions. Second lens 260 corrects the image displayed on display 40 for depth perception differences between the enhanced and non-enhanced images. Method 400 proceeds to step 430.
Step 430: Picking up catheter 300

In this step, the user, looking away from display 40 and, using his/her naked eye, picks up catheter 300 to be inserted into the target blood vessel, as described in step 425. Method 400 proceeds to step 435.
Step 435: Aligning catheter 300

In this step, the user, utilizing the enhanced image appearing on display 40, pulls the patient's skin tightly over the target blood vessel located in step 425 and aligns catheter 300 directly over and parallel to the target blood vessel. Method 400 proceeds to step 440.
Step 440: Inserting catheter 300 into target blood vessel

In this step, the user, by utilizing either his/her naked eye or the enhanced image appearing on display 40, pierces skin surface 225 with needle tip 360 and introduces the catheter 300 into the target blood vessel. During this process, catheter 300 becomes visible via display 40, which allows the user to determine the accuracy of the needle placement. US2004/0019280, US2003/0187360, and US2002/0115922 fully describes a system in which an IR-opaque or IR reflective substance or pattern is applied to cannula sheathing 320 , which makes the travel path of cannula sheathing 320 clearly visible to the user via display 40, so that the user may gauge its position and travel path more accurately. Alternatively, needle tip 360 may be doped with an IR-opaque or IR reflective substance or pattern, which makes the travel path of needle tip 360 clearly visible to the user via display 40, so that the user may gauge its position and travel path more accurately. When catheter 300 enters the target blood vessel, blood will flow into flash chamber 370. The user may confirm the entry of catheter needle 350 into the target blood vessel via display 40. By using the enhanced image of the target blood vessel displayed via display 40, the user may pierce the appropriate blood vessel more accurately and rapidly and, thus, save time and money and reduce the patient's physical and emotional pain and trauma. Method 400 proceeds to step 445.
Step 445: Advancing catheter 300

In this step, the user, by utilizing either his/her naked eye or the enhanced image that appears on display 40, advances catheter 300 into the target blood vessel until a sufficient depth of penetration has been reached. Method 400 proceeds to step 450.
Step 450: Withdrawing catheter needle 350 and catheter body 380

Catheter needle 350 and catheter body 380 are withdrawn leaving cannula 310 remaining in the target blood vessel. Cannula 310 is secured in place using standard medical practices. Method 400 proceeds to step 455.
Step 455: Completing procedure

In this step, the user completes the catheter 300 insertion procedure by using standard medical practices. This may include, but is not limited to, releasing the tourniquet, and attaching IV tubing to cannula housing 340, for example. Method 400 proceeds to step 460.
Step 460: Removing the headset 12

In this step, the user removes the headset 12 from his/her head and powers off the system 10. Alternatively, the user prepares additional patients/body target areas for imaging and catheterization. Method 400 ends.

Although the present invention has been described in considerable detail with reference to certain preferred versions thereof, these preferred versions are not to be understood in a way limiting the scope of the claims.

## Claims

1. A needle insertion system for inserting a needle within a blood vessel, said system comprising:
an imaging system (10) comprising:
at least one infrared emitter (32, 34) configured to illuminate a region under a surface of skin with waves of infrared light (215);
an infrared detector (38) configured to accept waves of infrared light (215) reflected from the region under the surface of the skin, said infrared detector (38) comprising an output for outputting a signal corresponding to unenhanced image data;
a computing unit comprising an input for accepting said unenhanced image data, a memory, means for enhancing and outputting result images in which enhanced images of blood vessels are shown within the images of the region under the surface of the skin, and an output for outputting said enhanced images in substantially real time;
a display device (40) for inputting said enhanced images from output of said computing unit and displaying said enhanced images; and
a power source in electrical communication with said infrared emitter (32, 34), said infrared detector (38), said computing unit and said display device; and a needle comprising a needle tip and a needle body;
wherein the elements of said system are adapted to fulfill the following functions: a user viewing said display of said imaging system (10) and locating a target blood vessel (220), aligning said needle body with said target blood vessel (220), piercing said blood vessel (220) with said needle tip, introducing said needle into said target blood vessel (220), advancing said needle until said display of said imaging system shows that a sufficient depth has been reached, performing a medical procedure, and withdrawing said needle from said blood vessel (220),
wherein the means for enhancing and outputting result images includes a digital processing unit programmed with computer program means for enhancing and outputting results images at a rate of at least five frames per second;
wherein said imaging system (10) further comprises a headset (12), wherein said at least one infrared emitter (32, 34), said infrared detector (38), said computing unit, said display (40), and said power source of said imaging system (10) are attached to said headset (12), and wherein said display (40) is disposed such that a user is able to view both said display and the surface of the skin without removing said headset (12);
wherein said display of said imaging system (10) comprises a pair of LCD screens and
wherein said imaging system (10) further comprises a pair of optical lenses (260) disposed between said LCD screens and a pair of eyes of a user.

2. The needle insertion system of claim 1, wherein said infrared detector (38) of said imaging system (10) comprises a CMOS camera (38) adapted to generate digital data corresponding to said waves of infrared light (215) reflected from the subcutaneous blood vessels located in the region under the surface of the skin.

3. The needle insertion system of claim 2 wherein said imaging system (10) further comprises a camera lens (240) disposed between the surface of the skin and said CMOS camera (38).

4. The needle insertion system of claim 1 wherein said computing unit of said imaging system (10) further comprises an interface (52) and wherein said means for enhancing and outputting result images comprises a digital signal processing unit (120).

5. The needle insertion system of claim 4 wherein said imaging system (10) further comprises a data input in communication with said digital signal processing unit (120) through said interface.

6. The needle insertion system of claim 1 wherein said imaging system (10) further comprises a data input (255), wherein said means for enhancing and outputting result images comprises a digital signal processing unit and wherein said data input (255) is in communication with said digital signal processing unit.

7. The needle insertion system of claim 1 wherein at least one substance for enhancing a visibility of said needle body by said imaging system (10) is disposed upon an outer surface of said needle body, wherein said at least one substance enhances a visibility of said needle body by said imaging system (10) when compared with a visibility of said needle body without said substance disposed thereon.

8. The needle insertion system of claim 7 wherein one of said at least one substance for enhancing a visibility of said needle body is an IR opaque substance.

9. The needle insertion system of claim 8 wherein said IR-opaque substance is disposed upon said outer surface of said needle body in a pattern that further enhances a visibility of said needle body by said imaging system (10) when compared with a visibility of said needle body without said IR-opaque substance disposed thereon in said pattern.

10. The needle insertion system of claim 7 wherein one of said at least one substance for enhancing a visibility of said needle body is an IR-reflective substance.

11. The needle insertion system of claim 10 wherein said IR-reflective substance is disposed upon said outer surface of said needle body in a pattern that further enhances a visibility of said catheter needle by said imaging system (10) when compared with a visibility of said needle body without said IR-reflective substance disposed thereon in said pattern.

12. The needle insertion system of claim 7 wherein said the needle insertion system comprises at least two substances, wherein one of said at least two substances for enhancing a visibility of said needle body is an IR-reflective substance and wherein one of said at least two substances for enhancing a visibility of said needle body is an IR-opaque substance.

13. The needle insertion system of claim 12 wherein said IR-reflective substance and said IR-opaque substance are each disposed upon an outer surface of said needle body in a pattern that further enhances a visibility of said needle by said imaging system (10) when compared with a visibility of said needle body without said IR-reflective substance and said IR-opaque substance disposed thereon in said pattern.

14. The needle insertion system of claim 1 wherein said needle is a manufactured of a molded plastic material and wherein at least one substance for enhancing a visibility of said needle body by said imaging system (10) is disposed within said plastic material, wherein said at least one substance enhances a visibility of said needle body by said imaging system when compared with a visibility of said needle body without said substance disposed within said plastic material.

15. The needle insertion system of claim 14 wherein said substance for enhancing a visibility of said needle body is an IR-opaque substance.

16. The needle insertion system of claim 15 wherein said IR-opaque substance is disposed within said plastic material in a pattern that further enhances a visibility of said needle body by said imaging system (10) when compared with a visibility of said needle body without said IR-opaque substance disposed therein in said pattern.

17. The needle insertion system of claim 14 wherein said substance for enhancing a visibility of said needle body is an IR-reflective substance.

18. The needle insertion system of claim 17 wherein said IR-reflective substance is disposed within said plastic material in a pattern that further enhances a visibility of said needle body by said imaging system (10) when compared with a visibility of said needle body without said IR-reflective substance disposed therein in said pattern.

19. The needle insertion system of claim 14 wherein the needle insertion system comprises at least two substances, wherein one of said at least two substances for enhancing a visibility of said needle body is an IR-reflective substance and wherein one of said at least two substances for enhancing a visibility of said needle body is an IR-opaque substance.

20. The needle insertion system of claim 19 wherein said IR-reflective substance and said IR-opaque substance are each disposed within said plastic material in a pattern that further enhances a visibility of said needle by said imaging system (10) when compared with a visibility of said needle body without said IR-reflective substance and said IR-opaque substance disposed thereon in said pattern.

21. The needle insertion system of claim 19 further comprising a catheter comprising a catheter body and a cannula, wherein said needle is a catheter needle attached to said catheter body.

22. The needle insertion system of claim 21 wherein at least one substance for enhancing a visibility of said needle body by said imaging system (10) is disposed upon an outer surface of said needle body, wherein said at least one substance enhances a visibility of said needle body by said imaging system (10) when compared with a visibility of said needle body without said substance disposed thereon.

23. The needle insertion system of claim 22 wherein one of said at least one substance for enhancing a visibility of said needle body is an IR opaque substance.

24. The needle insertion system of claim 23 wherein said IR-opaque substance is disposed upon said outer surface of said needle body in a pattern that further enhances a visibility of said needle body by said imaging system (10) when compared with a visibility of said needle body without said IR-opaque substance disposed thereon in said pattern.

25. The needle insertion system of claim 22 wherein one of said at least one substance for enhancing a visibility of said needle body is an IR-reflective substance.

26. The needle insertion system of claim 25 wherein said IR-reflective substance is disposed upon said outer surface of said needle body in a pattern that further enhances a visibility of said catheter needle by said imaging system (10) when compared with a visibility of said needle body without said IR-reflective substance disposed thereon in said pattern.

27. The needle insertion system of claim 22 wherein the needle insertion system comprises at least two substances, wherein one of said at least two substances for enhancing a visibility of said needle body is an IR-reflective substance and wherein one of said at least two substances for enhancing a visibility of said needle body is an IR-reflective substance.

28. The needle insertion system of claim 27 wherein said IR-reflective substance and said IR-opaque substance are each disposed upon an outer surface of said needle body in a pattern that further enhances a visibility of said needle by said imaging system (10) when compared with a visibility of said needle body without said IR-reflective substance and said IR-opaque substance disposed thereon in said pattern.

## Patentansprüche

1. Nadeleinführungssystem zum Einführen einer Nadel in ein Blutgefäß, wobei das System umfasst:
ein Bildgebungssystem (10), umfassend:
mindestens einen Infrarotemitter (32, 34), der zum beleuchten einer Region unter der Hautoberfläche mit Infrarotlichtwellen (215) konfiguriert ist;
einen Infrarotdetektor (38), der zum Aufnehmen von von der Region unter der Hautoberfläche reflektierten Infrarotlichtwellen konfiguriert ist, wobei der Infrarotdetektor (38) eine Ausgabe zum Ausgeben eines unverbesserten Bilddaten entsprechenden Signals umfasst;
eine Recheneinheit, umfassend eine Eingabe zum Aufnehmen der unverbesserten Bilddaten, einen Speicher, ein Mittel zum Verbessern und Ausgeben von erhaltenen Bildern, wobei verbesserte Bilder von Blutgefäßen in den Bildern der Region unter der Hautoberfläche gezeigt werden, und eine Ausgabe zum Ausgeben der verbesserten Bilder im Wesentlichen in Echtzeit;
eine Anzeigevorrichtung (40) zum Eingeben der verbesserten Bilder von der Ausgabe der Recheneinheit und Anzeigen der verbesserten Bilder; und
eine Stromquelle in elektrischer Kommunikation mit dem Infrarotemitter (32, 34), dem Infrarotdetektor (38), der Recheneinheit und der Anzeigevorrichtung;
und eine eine Nadelspitze und einen Nadelkörper umfassende Nadel;
wobei die Elemente des Systems zum Erfüllen der folgenden Funktionen angepasst sind: Ein Anwender betrachtet die Anzeige des Bildgebungssystems (10) und lokalisiert das Zielblutgefäß (220), richtet den Nadelköper an dem Blutgefäß (220) aus, Sticht mit der Nadelspitze in das Blutgefäß (220), führt die Nadel in das Blutgefäß (220) ein, bewegt die Nadel weiter, bis die Anzeige des Bildgebungssystems zeigt, dass eine ausreichende Tiefe erreicht wurde, führt eine medizinische Prozedur durch und zieht die Nadel aus dem Blutgefäß (220) heraus,
wobei das Mittel zum Verbessern und Ausgeben von erhaltenen Bildern eine digitale Verarbeitungseinheit einschließt, die mit Computerprogrammmittel zum Verbessern und Ausgeben von erhaltenen Bildern mit einer Geschwindigkeit von mindestens fünf Frames pro Sekunde programmiert ist;
wobei das Bildgebungssystem (10) des Weiteren ein Headset (12) umfasst, wobei der mindestens eine Infrarotemitter (32, 34), der Infrarotdetektor (38), die Recheneinheit, die Anzeige (40) und die Stromquelle des Bildgebungssystems (10) an dem Headset (12) angebracht sind, und wobei die Anzeige (40) derart angeordnet ist, dass ein Anwender in der Lage ist, sowohl die Anzeige als auch die Hautoberfläche ohne Entfernen des Headsets (12) zu betrachten;
wobei die Anzeige des Bildgebungssystems (10) ein Paar LCD-Bildschirme umfasst und
wobei das Bildgebungssystem (10) des Weiteren ein Paar optische Linsen (260) umfasst, die zwischen den LCD-Bildschirmen und dem Augenpaar eines Anwenders angeordnet werden.

2. Nadeleinführungssystem nach Anspruch 1, wobei der Infrarotdetektor (38) des Bildgebungssystems (10) eine CMOS-Kamera (38) umfasst, die dazu angepasst ist, digitale Daten zu erzeugen, die den Infrarotlichtwellen (215) entsprechen, die von in der Region unter der Hautoberfläche lokalisierten subkutanen Blutgefäßen reflektiert werden.

3. Nadeleinführungssystem nach Anspruch 2, wobei das Bildgebungssystem (10) des Weiteren eine Kameralinse (240) umfasst, die zwischen der Hautoberfläche und der CMOS-Kamera (38) angeordnet wird.

4. Nadeleinführungssystem nach Anspruch 1, wobei die Recheneinheit des Bildgebungssystems (10) des Weiteren eine Schnittstelle (52) aufweist und wobei das Mittel zum Verbessern und Ausgeben von erhaltenen Bildern eine digitale Signalverarbeitungseinheit (120) umfasst.

5. Nadeleinführungssystem nach Anspruch 4, wobei das Bildgebungssystem (10) des Weiteren eine Dateneingabe in Kommunikation mit der digitalen Signalverarbeitungseinheit (120) durch die Schnittstelle umfasst.

6. Nadeleinführungssystem nach Anspruch 1, wobei das Bildgebungssystem (10) des Weiteren eine Dateneingabe (255) umfasst, wobei das Mittel zum Verbessern und Ausgeben von erhaltenen Bildern eine digitale Signalverarbeitungseinheit umfasst, und wobei die Dateneingabe (255) in Kommunikation mit der digitalen Signalverarbeitungseinheit ist.

7. Nadeleinführungssystem nach Anspruch 1, wobei mindestens eine Substanz zum Verbessern der Sichtbarkeit des Nadelkörpers durch das Bildgebungssystems (10) auf einer Außenfläche des Nadelkörpers angeordnet ist, wobei die mindestens eine Substanz die Sichtbarkeit des Nadelkörpers durch das Bildgebungssystem (10) verglichen mit der Sichtbarkeit des Nadelkörpers ohne die darauf angeordnete Substanz verbessert.

8. Nadeleinführungssystem nach Anspruch 7, wobei eine der mindestens einen Substanz zum Verbessern der Sichtbarkeit des Nadelkörpers eine IR-undurchlässige Substanz ist.

9. Nadeleinführungssystem nach Anspruch 8, wobei die IR-undurchlässige Substanz auf der Außenfläche des Nadelkörpers in einem Muster angeordnet ist, das die Sichtbarkeit des Nadelkörpers durch das Bildgebungssystem (10) verglichen mit der Sichtbarkeit des Nadelkörpers ohne die darauf in dem Muster angeordnete IR-undurchlässige Substanz weiter verbessert.

10. Nadeleinführungssystem nach Anspruch 7, wobei eine der mindestens einen Substanz zum Verbessern der Sichtbarkeit des Nadelkörpers eine IR-reflektierende Substanz ist.

11. Nadeleinführungssystem nach Anspruch 10, wobei die IR-reflektierende Substanz auf der Außenfläche des Nadelkörpers in einem Muster angeordnet ist, das die Sichtbarkeit des Nadelkörpers durch das Bildgebungssystem (10) verglichen mit der Sichtbarkeit des Nadelkörpers ohne die darauf in dem Muster angeordnete IR-reflektierende Substanz weiter verbessert.

12. Nadeleinfiihrungssystem nach Anspruch 7, wobei das Nadeleinführungssystem mindestens zwei Substanzen umfasst, wobei es sich bei einer der mindestens zwei Substanzen zum Verbessern der Sichtbarkeit des Nadelkörpers um eine IR-reflektierende Substanz handelt, und wobei es sich bei einer der mindestens zwei Substanzen zum Verbessern der Sichtbarkeit des Nadelkörpers um eine IR-undurchlässige Substanz handelt.

13. Nadeleinfiihrungssystem nach Anspruch 12, wobei die IR-reflektierende Substanz und die IR-undurchlässige Substanz jeweils auf einer Außenfläche des Nadelkörpers in einem Muster angeordnet sind, das die Sichtbarkeit des Nadelkörpers durch das Bildgebungssystem (10) verglichen mit der Sichtbarkeit des Nadelkörpers ohne die darauf in dem Muster angeordnete IR-reflektierende Substanz und IR-undurchlässige Substanzweiter verbessert.

14. Nadeleinführungssystem nach Anspruch 1, wobei die Nadel aus einem geformten Kunststoffmaterial hergestellt ist und wobei mindestens eine Substanz zum Verbessern der Sichtbarkeit des Nadelkörpers durch das Bildgebungssystem (10) in dem Kunststoffmaterial angeordnet ist, wobei mindestens eine Substanz die Sichtbarkeit des Nadelkörpers durch das Bildgebungssystem verglichen mit der Sichtbarkeit des Nadelkörpers ohne die im Kunststoffmaterial angeordnete Substanz verbessert.

15. Nadeleinfiihrungssystem nach Anspruch 14, wobei die Substanz zum Verbessern der Sichtbarkeit des Nadelkörpers eine IR-undurchlässige Substanz ist.

16. Nadeleinführungssystem nach Anspruch 15, wobei die IR-undurchlässige Substanz in dem Kunststoffmaterial in einem Muster angeordnet ist, das die Sichtbarkeit des Nadelkörpers durch das Bildgebungssystem (10) verglichen mit der Sichtbarkeit des Nadelkörpers ohne die in dem Kunststoffmaterial in dem Muster angeordnete IR-undurchlässige Substanz weiter verbessert.

17. Nadeleinführungssystem nach Anspruch 14, wobei die Substanz zum Verbessern der Sichtbarkeit des Nadelkörpers eine IR-reflektierende Substanz ist.

18. Nadeleinführungssystem nach Anspruch 17, wobei die IR-reflektierende Substanz in dem Kunststoffmaterial in einem Muster angeordnet ist, das die Sichtbarkeit des Nadelkörpers durch das Bildgebungssystem (10) verglichen mit der Sichtbarkeit des Nadelkörpers ohne die darin in dem Muster angeordnete IR-reflektierende Substanz weiter verbessert.

19. Nadeleinführungssystem nach Anspruch 14, wobei das Nadeleinführungssystem mindestens zwei Substanzen umfasst, wobei es sich bei einer der mindestens zwei Substanzen zum Verbessern der Sichtbarkeit des Nadelkörpers um eine IR-reflektierende Substanz handelt, und wobei es sich bei einer der mindestens zwei Substanzen zum Verbessern der Sichtbarkeit des Nadelkörpers um eine IR-undurchlässige Substanz handelt.

20. Nadeleinführungssystem nach Anspruch 19, wobei die IR-reflektierende und die IR-undurchlässige Substanz jeweils in dem Kunststoffinaterial in einem Muster angeordnet sind, das die Sichtbarkeit des Nadelkörpers durch das Bildgebungssystem (10) verglichen mit der Sichtbarkeit des Nadelkörpers ohne die darin in dem Muster angeordnete IR -reflektierende und IR-undurchlässige Substanz weiter verbessert.

21. Nadeleinführungssystem nach Anspruch 19, des Weiteren umfassend einen Katheder, der einen Katheterkörper und eine Kanüle umfasst, wobei die Nadel eine an dem Katheterkörper angebrachte Katheternadel ist.

22. Nadeleinführungssystem nach Anspruch 21, wobei mindestens eine Substanz zum Verbessern der Sichtbarkeit des Nadelkörpers durch das Bildgebungssystems (10) auf einer Außenfläche des Nadelkörpers angeordnet ist, wobei die mindestens eine Substanz die Sichtbarkeit des Nadelkörpers durch das Bildgebungssystem (10) verglichen mit der Sichtbarkeit des Nadelkörpers ohne die darauf angeordnete Substanz verbessert.

23. Nadeleinführungssystem nach Anspruch 22, wobei eine der mindestens einen Substanz zum Verbessern der Sichtbarkeit des Nadelkörpers eine IR-undurchlässige Substanz ist.

24. Nadeleinführungssystem nach Anspruch 23, wobei die IR-undurchlässige Substanz auf der Außenfläche des Nadelkörpers in einem Muster angeordnet ist, das die Sichtbarkeit des Nadelkörpers durch das Bildgebungssystem (10) verglichen mit der Sichtbarkeit des Nadelkörpers ohne die darauf in dem Muster angeordnete IR-undurchlässige Substanz weiter verbessert.

25. Nadeleinführungssystem nach Anspruch 22, wobei eine der mindestens einen Substanz zum Verbessern der Sichtbarkeit des Nadelkörpers eine IR-reflektierende Substanz ist.

26. Nadeleinführungssystem nach Anspruch 26, wobei die IR-reflektierende Substanz auf der Außenfläche des Nadelkörpers in einem Muster angeordnet ist, das die Sichtbarkeit des Nadelkörpers durch das Bildgebungssystem (10) verglichen mit der Sichtbarkeit des Nadelkörpers ohne die darauf in dem Muster angeordnete IR-reflektierende Substanz weiter verbessert.

27. Nadeleinführungssystem nach Anspruch 22, wobei das Nadeleinführungssystem mindestens zwei Substanzen umfasst, wobei es sich bei einer der mindestens zwei Substanzen zum Verbessern der Sichtbarkeit des Nadelkörpers um eine IR-reflektierende Substanz handelt, und wobei es sich bei einer der mindestens zwei Substanzen zum Verbessern der Sichtbarkeit des Nadelkörpers um eine IR-undurchlässige Substanz handelt.

28. Nadeleinführungssystem nach Anspruch 27, wobei die IR-reflektierende Substanz und die IR-undurchlässige Substanz jeweils auf einer Außenfläche des Nadelkörpers in einem Muster angeordnet sind, das die Sichtbarkeit des Nadelkörpers durch das Bildgebungssystem (10) verglichen mit der Sichtbarkeit des Nadelkörpers ohne die darauf in dem Muster angeordnete IR-reflektierende Substanz und die IR-undurchlässige Substanzweiter verbessert.

## Revendications

1. Un système d'insertion d'aiguille pour insérer une aiguille dans un vaisseau sanguin, ledit système comprenant :
un système d'imagerie (10) comprenant :
au moins un émetteur infrarouge (32, 34) configuré pour illuminer une région sous une surface de la peau par des ondes de lumière infrarouge (215) ;
un détecteur infrarouge (38) configuré pour recevoir des ondes de lumière infrarouge (215) réfléchies depuis la région sous la surface de la peau, ledit détecteur infrarouge (38) comprenant une sortie pour délivrer en sortie un signal correspondant à des données d'images non renforcées ;
une unité de calcul comprenant une entrée pour recevoir lesdites données d'images non renforcées, une mémoire, des moyens pour renforcer et délivrer en sortie des images résultantes dans lesquelles des images renforcées des vaisseaux sanguins sont représentées dans des images de la région sous la surface de la peau, et une sortie pour délivrer en sortie lesdites images renforcées pratiquement en temps réel ;
un dispositif d'affichage (40) pour appliquer en entrée lesdites images renforcées provenant de la sortie de ladite unité de calcul et afficher lesdites images renforcées ; et
une source d'alimentation en communication électrique avec ledit émetteur infrarouge (32, 34), ledit détecteur infrarouge (38), ladite unité de calcul et ledit dispositif d'affichage ;
et une aiguille comprenant une extrémité d'aiguille et un corps d'aiguille ;
dans lequel les éléments dudit système sont aptes à remplir les fonctions suivantes : un utilisateur visualisant ledit afficheur dudit système d'imagerie (10) et localisant un vaisseau sanguin cible (220), alignant ledit corps d'aiguille avec ledit vaisseau sanguin cible (220), perçant ledit vaisseau sanguin cible (220) avec ladite extrémité d'aiguille, introduisant ladite aiguille dans ledit vaisseau sanguin cible (220), faisant progresser ladite aiguille jusqu'à ce que ledit afficheur dudit système d'imagerie montre qu'une profondeur suffisante a été atteinte, exécutant une procédure médicale, et retirant ladite aiguille dudit vaisseau sanguin (220),
dans lequel les moyens de renforcement et de délivrance en sortie d'images résultantes inclut une unité de traitement numérique programmée par des moyens de programme informatique pour renforcer et délivrer en sortie des images résultantes à une cadence d'au moins cinq trames par seconde ;
dans lequel ledit système d'imagerie (10) comprend en outre un casque (12), ledit au moins un émetteur infrarouge (32, 34), ledit détecteur infrarouge (38), ladite unité de calcul, ledit afficheur (40) et ladite source d'alimentation dudit système d'imagerie (10) sont fixés audit casque (12), et dans lequel ledit afficheur (40) est disposé de telle manière qu'un utilisateur soit capable de visualiser à la fois ledit afficheur et la surface de la peau sans retirer ledit casque (12) ;
dans lequel ledit afficheur dudit système d'imagerie (10) comprend deux écrans LCD, et
dans lequel ledit système d'imagerie (10) comprend en outre deux lentilles optiques (260) disposées entre lesdits écrans LCD et deux yeux d'un utilisateur.

2. Le système d'insertion d'aiguille de la revendication 1, dans lequel ledit détecteur infrarouge (38) dudit système d'imagerie (10) comprend une caméra CMOS (38) apte à générer des données numériques correspondant auxdites ondes de lumière infrarouge (215) réfléchies depuis les vaisseaux sanguins subcutanés situés dans la région sous la surface de la peau.

3. Le système d'insertion d'aiguille de la revendication 2, dans lequel ledit système d'imagerie (10) comprend en outre un objectif de caméra (240) disposé entre la surface de la peau et ladite caméra CMOS (38).

4. Le système d'insertion d'aiguille de la revendication 1, dans lequel ladite unité de calcul dudit système d'imagerie (10) comprend en outre une interface (52) et dans lequel lesdits moyens de renforcement et de délivrance en sortie d'images résultantes comprennent une unité de traitement de signal numérique (120).

5. Le système d'insertion d'aiguille de la revendication 4, dans lequel ledit système d'imagerie (10) comprend en outre une entrée de données en communication avec ladite unité de traitement de signal numérique (120) via ladite interface.

6. Le système d'insertion d'aiguille de la revendication 1, dans lequel ledit système d'imagerie (10) comprend en outre une entrée de données (255), lesdits moyens de renforcement et de délivrance en sortie d'images résultantes comprenant une unité de traitement de signal numérique et dans lequel ladite entrée de données (255) est en communication avec ladite unité de traitement de signal numérique.

7. Le système d'insertion d'aiguille de la revendication 1, dans lequel au moins une substance de renforcement d'une visibilité dudit corps d'aiguille par ledit système d'imagerie (10) est disposée sur une surface extérieure dudit corps d'aiguille, ladite au moins une substance renforçant une visibilité dudit corps d'aiguille par ledit système d'imagerie (10) par rapport à une visibilité dudit corps d'aiguille sans substance disposée dessus.

8. Le système d'insertion d'aiguille de la revendication 7, dans lequel l'une desdites au moins une substance de renforcement d'une visibilité dudit corps d'aiguille est une substance opaque aux IR.

9. Le système d'insertion d'aiguille de la revendication 8, dans lequel ladite substance opaque aux IR est disposée sur ladite surface extérieure dudit corps d'aiguille avec un motif qui renforce encore plus une visibilité dudit corps d'aiguille par ledit système d'imagerie (10) par rapport à une visibilité dudit corps d'aiguille sans ladite surface opaque aux IR disposée dessus avec ledit motif.

10. Le système d'insertion d'aiguille de la revendication 7, dans lequel l'une desdites au moins une substance de renforcement d'une visibilité dudit corps d'aiguille est une substance réfléchissant les IR.

11. Le système d'insertion d'aiguille de la revendication 10, dans lequel ladite substance réfléchissant les IR est disposée sur ladite surface extérieure dudit corps d'aiguille avec un motif qui renforce encore plus une visibilité de ladite aiguille de cathéter par ledit système d'imagerie (10) par rapport à une visibilité dudit corps d'aiguille sans ladite substance réfléchissant les IR disposée dessus avec ledit motif.

12. Le système d'insertion d'aiguille de la revendication 7, dans lequel le système d'insertion d'aiguille comprend au moins deux substances, l'une desdites au moins deux substances pour renforcer une visibilité dudit corps d'aiguille étant une substance réfléchissant les IR et l'une desdites au moins deux substances de renforcement d'une visibilité dudit corps d'aiguille étant une substance opaque aux IR.

13. Le système d'insertion d'aiguille de la revendication 12, dans lequel ladite substance réfléchissant les IR et ladite substance opaque aux IR sont disposées chacune sur une surface extérieure dudit corps d'aiguille avec un motif qui renforce encore plus une visibilité dudit corps d'aiguille par ledit système d'imagerie (10) par rapport à une visibilité dudit corps d'aiguille sans ladite substance réfléchissant les IR et ladite substance opaque aux IR disposée dessus avec ledit motif.

14. Le système d'insertion d'aiguille de la revendication 1, dans lequel ladite aiguille est fabriquée à partir d'une matière plastique moulée et dans lequel au moins une substance de renforcement d'une visibilité dudit corps d'aiguille par ledit système d'imagerie (10) est disposée au sein de ladite matière plastique, ladite au moins une substance renforçant une visibilité dudit corps d'aiguille par ledit système d'imagerie par rapport à une visibilité dudit corps d'aiguille sans ladite substance disposée au sein de ladite matière plastique.

15. Le système d'insertion d'aiguille de la revendication 14, dans lequel ladite substance de renforcement d'une visibilité dudit corps d'aiguille est une substance opaque aux IR.

16. Le système d'insertion d'aiguille de la revendication 15, dans lequel ladite substance opaque aux IR est disposée au sein de ladite matière plastique avec un motif qui renforce encore plus une visibilité dudit corps d'aiguille par ledit système d'imagerie (10) par rapport à une visibilité dudit corps d'aiguille sans ladite substance opaque aux IR disposée dedans avec ledit motif.

17. Le système d'insertion d'aiguille de la revendication 14, dans lequel ladite substance de renforcement d'une visibilité dudit corps d'aiguille est une substance réfléchissant les IR.

18. Le système d'insertion d'aiguille de la revendication 17, dans lequel ladite substance réfléchissant les IR est disposée au sein de ladite matière plastique avec un motif qui renforce encore plus une visibilité dudit corps d'aiguille par ledit système d'imagerie par rapport à une visibilité dudit corps d'aiguille sans ladite substance réfléchissant les IR disposée dedans avec ledit motif.

19. Le système d'insertion d'aiguille de la revendication 14, dans lequel le système d'insertion d'aiguille comprend au moins deux substances, l'une desdites au moins deux substances de renforcement d'une visibilité dudit corps d'aiguille étant une substance réfléchissant les IR et l'une desdites au moins deux substances de renforcement d'une visibilité dudit corps d'aiguille étant une substance opaque aux IR.

20. Le système d'insertion d'aiguille de la revendication 19, dans lequel ladite substance réfléchissant les IR et ladite substance opaque aux IR sont disposées chacune au sein de ladite matière plastique avec un motif qui renforce encore plus une visibilité de ladite aiguille par ledit système d'imagerie (10) par rapport à une visibilité dudit corps d'aiguille sans ladite substance réfléchissant l'IR et ladite substance opaque aux IR disposée dessus avec ledit motif.

21. Le système d'insertion d'aiguille de la revendication 19, comprenant en outre un cathéter comprenant un corps de cathéter et une canule, ladite aiguille étant une aiguille de cathéter solidarisée audit corps de cathéter.

22. Le système d'insertion d'aiguille de la revendication 21, dans lequel au moins une substance de renforcement d'une visibilité dudit corps d'aiguille par ledit système d'imagerie (10) est disposée sur une surface extérieure dudit corps d'aiguille, dans lequel ladite au moins une substance renforce une visibilité dudit corps d'aiguille par ledit système d'imagerie (10) par rapport à une visibilité dudit corps d'aiguille sans ladite substance disposée dessus.

23. Le système d'insertion d'aiguille de la revendication 22, dans lequel l'une desdites au moins une substance de renforcement d'une visibilité dudit corps d'aiguille est une substance opaque aux IR.

24. Le système d'insertion d'aiguille de la revendication 23, dans lequel ladite substance opaque aux IR est disposée sur ladite surface extérieure dudit corps d'aiguille avec un motif qui renforce encore plus une visibilité dudit corps d'aiguille par ledit système d'imagerie (10) par rapport à une visibilité dudit corps d'aiguille sans ladite substance opaque aux IR disposée dessus avec ledit motif.

25. Le système d'insertion d'aiguille de la revendication 22, dans lequel l'une desdites au moins une substance de renforcement d'une visibilité dudit corps d'aiguille est une substance réfléchissant les IR.

26. Le système d'insertion d'aiguille de la revendication 25, dans lequel ladite substance réfléchissant les IR est disposée sur ladite surface extérieure dudit corps d'aiguille avec un motif qui renforce encore plus une visibilité de ladite aiguille de cathéter par ledit système d'imagerie (10) par rapport à une visibilité dudit corps d'aiguille sans ladite substance réfléchissant les IR disposée dessus avec ledit motif.

27. Le système d'insertion d'aiguille de la revendication 22, dans lequel le système d'insertion d'aiguille comprend au moins deux substances, l'une desdites au moins deux substances de renforcement d'une visibilité dudit corps d'aiguille étant une substance réfléchissant les IR et l'une desdites au moins deux substances de renforcement d'une visibilité dudit corps d'aiguille étant une substance réfléchissant les IR.

28. Le système d'insertion d'aiguille de la revendication 27, dans lequel ladite substance réfléchissant les IR et ladite substance opaque aux IR sont disposées chacune sur une surface extérieure dudit corps d'aiguille avec un motif qui renforce encore plus une visibilité de ladite aiguille par ledit système d'imagerie (10) par rapport à une visibilité dudit corps d'aiguille sans ladite substance réfléchissant les IR et ladite substance opaque aux IR disposée dessus avec ledit motif.
